(19) Europäisches Patentamt
European Patent Office
Office européen des brevets

(11) EP 4 591 788 B1

(12) EUROPEAN PATENT SPECIFICATION

(45) Date of publication and mention
of the grant of the patent:
08.04.2026 Bulletin 2026/15

(21) Application number: 24154214.1

(22) Date of filing: 26.01.2024

(51) International Patent Classification (IPC):
**A61B 5/087** [(2006.01)]

(52) Cooperative Patent Classification (CPC):
**A61B 5/087;** A61B 2560/0223; A61B 2562/0219

(54) **ASSEMBLY COMPRISING A DEVICE FOR MEASURING A FLOW OF GAS AND METHOD FOR VERIFYING A CALIBRATION OF SUCH A DEVICE**

ANORDNUNG MIT EINER VORRICHTUNG ZUR MESSUNG EINES GASSTROMS UND VERFAHREN ZUR ÜBERPRÜFUNG EINER KALIBRIERUNG SOLCH EINER VORRICHTUNG

ENSEMBLE COMPRENANT UN DISPOSITIF DE MESURE D'UN FLUX DE GAZ ET PROCÉDÉ DE VÉRIFICATION D'UN ÉTALONNAGE D'UN TEL DISPOSITIF

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
30.07.2025 Bulletin 2025/31

(73) Proprietor: **ndd Medizintechnik AG**
**8005 Zürich (CH)**

(72) Inventors:
• **ZHUANG, Katie**
**8055 Zurich (CH)**

• **BUESS, Christian**
**8810 Horgen (CH)**
• **DANERS, Felix**
**8200 Schaffhausen (CH)**

(74) Representative: **Maikowski & Ninnemann**
**Patentanwälte Partnerschaft mbB**
**Postfach 15 09 20**
**10671 Berlin (DE)**

(56) References cited:
CN-A- 109 009 130          US-A1- 2015 369 795
US-A1- 2019 254 534      US-A1- 2021 338 104
US-B2- 7 367 955

## Description

The present invention relates to an assembly comprising a device for measuring a flow of gas according to claim 1 and to a method for verifying a calibration of a device for measuring a flow of gas according to claim 10. Further aspects of the invention are defined in dependent claims.

Devices for measuring the flow of gas are used in many applications such as industrial or medical applications. Spirometers, i.e., devices that are used to assess lung function in patients, are an example of such devices for measuring the flow of gas. For that purpose, spirometers measure the air flow in and out of the patient's lungs. Air flow is measured, and volume is calculated from air flow by integrating over time. Hence, the air flow measurement must be highly accurate, and the calibration of the spirometer must always be guaranteed.

The accuracy of spirometers is typically verified using a 3-liter calibration syringe. For that purpose, a known volume of air is passed through the spirometer at varying speeds to verify that the spirometer measures a 3-liter volume independent of the flow speed used. This procedure is also proposed in international standards of the American Thoracic Society (ATS) and the European Respiratory Society (ERS). In the 'Standardization of Spirometry 2019 Update' (Graham BL, Steenbruggen I, Miller MR, et al. Standardization of Spirometry 2019 Update. Am J Respir Crit Care Med 2019;200(8):e70-e88) a daily calibration check using a calibration syringe is suggested.

Such a daily calibration check is cumbersome. In addition, manual handling of a 3-liter calibration syringe is often connected with handling errors so that the calibration check does not provide the intended accuracy.

EP 3 566 647 A1 describes a method for verifying a calibration of an ultrasonic spirometer, the method comprising the following steps: a) determining an actual value of a distance between a first ultrasonic transducer and a second ultrasonic transducer of a spirometer; b) determining a difference between the actual value of the distance and a nominal value of the distance that is assigned to the spirometer; and c1) accepting an actual calibration of the spirometer if an absolute value of the difference is smaller than or equal to a first threshold value, or c2) refusing an actual calibration of the spirometer if an absolute value of the difference is bigger than the first threshold value, wherein the first threshold value is 5 % of the nominal value of the distance.

CN 109009130 A describes a lung function instrument having a sensor handle and featuring a shaking and direction detection. The sensor handle comprises a pressure difference sensor and a tri-axial acceleration sensor. In a lung function test process, it is tested whether the sensor handle is located in a test position. In addition, the shaking condition of the sensor handle can be monitored by employing the tri-axial acceleration sensor so that the accuracy of the flow baseline of the lung function instrument is improved and the detection error is reduced.

US 2019/0254534 A1 describes a spirometer comprising a MEMS-based (MEMS; microelectromechanical systems) thermal fluid flow sensor for generating a signal in response to a fluid flow generated during inhalation or exhalation; and a microcontroller for calculating the fluid flow from the signal generated by the flow sensor. The spirometer may be connected to other devices, such as a smartphone or a personal computer or any other computing unit which is adapted to collect, store, analyze, exchange and/or display data. The spirometer may further comprise an acceleration sensor which is different from the MEMS-based thermal fluid flow sensor. The microcontroller of the spirometer may be programmed to calculate a corrected fluid flow from the signal generated by the flow sensor and from a signal generated by the acceleration sensor. Furthermore, this acceleration sensor may also be employed - similar to the MEMS-based thermal fluid flow sensor - for measuring the temperature of the breath. US 2021/338104 A1 describes calibration of a device for measuring a flow of gas by providing a calibration volume of gas with a syringe.

It is an object of the present invention to provide an assembly with a device for measuring a flow of gas that enables an easier calibration verification than the devices known from prior art. This object is achieved with an assembly comprising a device for measuring the flow of gas having the claim elements of claim 1. Such a device comprises a gas inlet, a gas outlet, and a flow sensor arranged between the gas inlet and the gas outlet. The gas inlet and/or the gas outlet have, in an embodiment, an open (unobstructed) construction without any flaps or obstacles that could hinder a flow of gas through the gas inlet or the gas outlet, respectively. The term "flow" as used herein refers to the flow rate Q, i.e., to the volume of gas which passes per unit time through a flow channel extending between the gas inlet and the gas outlet ( $Q = \dot{V} = \frac{dV}{dt}$ ). The flow or flow rate can also be referred to as "volumetric flow rate", "volume flow rate", or "volume velocity". Furthermore, the gas flow or flow of gas can also be designated as "gas flow velocity" or "average gas flow velocity in the flow channel".

The assembly further comprises a motion sensor. The motion sensor is a device that is able to detect a position of the device for measuring the flow of gas in space over time. Expressed in other words, the motion sensor serves for tracking a motion of the device as the device is moved through the space surrounding the device, such as air. The obtained motion data is used to calculate the flow of gas that has passed through the device for measuring the flow of gas due to the movement of the device. By comparing the calculated flow of gas with the measured flow of gas, a previously performed calibration of the device can be verified. Expressed in other words, the device enables a simple verification of the accuracy or verification of calibration by tracking movements of the device.

The assembly, in particular the device, comprises a processor and a memory unit or is operatively connected with a processor and with memory unit. In this context, the memory unit comprises computer-readable code that causes the processor to perform the steps explained in the following when being executed by the processor.

In a first step, a movement of the device through a space surrounding the device is detected with the motion sensor. In another method step, a gas flow through the device during the movement of the device is detected with the flow sensor. Additionally, a first characteristic value that represents or is based on the detected gas flow is determined with the processor. In a further method step, a second characteristic value that represents or is based on an expected gas flow that corresponds to the detected movement of the device is determined with the processor. In a further method step, the second characteristic value is compared with the first characteristic value. In addition, an absolute value of a difference between the second characteristic value and the first characteristic value is determined.

In a further method step, a first signal is generated if a first condition is met, and a second signal is generated if a second condition is met. According to the first condition, the absolute value of the difference between the second characteristic value and the first characteristic value is smaller than a predeterminable threshold. According to the second condition, the absolute value of the difference between the second characteristic value and the first characteristic value is equal to or larger than a predeterminable threshold. If the first condition is met, the generated first signal indicates that the calibration of the device still lies within a predeterminable range and is considered to be in order. If the second condition is met, the generated second signal indicates that the calibration of the device is no longer in order. In such a case, the calibration needs to be repeated or the device needs to be repaired/recalibrated. Expressed in other words, the second signal generated if the second condition is met indicates that the device is out of calibration. Thus, if the second condition is met, the user can, e.g., contact a support center for the device and send the device to a service provider or the manufacturer to obtain a repair or recalibration of the device.

Thus, it is no longer necessary to perform a calibration verification with a 3-liter syringe (or another syringe) so that the calibration verification that needs to be performed on a regular basis is simplified. In addition, the device does not require specific movements on a predefined trajectory to achieve calibration verification. Rather, a random movement is fully sufficient to obtain data allowing a reliable calibration verification, provided that the movement results in the device being moved through space such that gas is passed from the gas inlet and/or the gas outlet through the device. Since the calibration verification by tracking the movements of the device with its motion sensor is particularly simple for a user, it is to be assumed that the calibration verification will be performed by more users on a regular basis, such as daily, as proposed in the above-mentioned "Standardization of Spirometry 2019 Update". This enhances the overall accuracy of spirometry measurements since many users do not perform a daily calibration check as proposed by the "Standardization of Spirometry 2019 Update" due to the complexity of the calibration verification process with a 3-liter syringe.

Summarizing, providing an assembly comprising a device for measuring the flow of gas and a motion sensor is a surprisingly simple way of enabling a novel method of calibration verification that is extremely user friendly, easy to handle and highly reliable.

In an embodiment, the motion sensor is arranged on a housing of the device. By placing the motion sensor on the housing of the device (such as on the surface of the housing of the device), it is very easy to equip devices for measuring the flow of gas with a motion sensor without requiring laborious new constructions of such housings. However, the motion sensor might be more exposed to damaging influences from outside the housing of the device. In an embodiment, the motion sensor is arranged within a housing of the device. Then, it is better protected against environmental influences acting upon the housing of the device than in case of placing the motion sensor on an (outer) surface of the housing.

If the motion sensor is placed on or within the housing of the device, it forms an integral part of the device. The assembly then comprises a single entity composed of a plurality of components.

In an embodiment, the motion sensor is arranged in a motion sensor unit that is separate from the device. In an embodiment, the motion sensing unit serves as cradle or base station for the device (e.g., for storing the device in a defined position, for charging a battery contained in the device, and/or for establishing a data connection to a different device connected to the base station so that the base station would additionally act as docking station). During operation of the assembly, it is only necessary that the motion sensor is able to track the movement of the device through space, e.g., by an optical measurement. This condition is fulfilled if the device is moved in front of the motion sensor unit without any obstacles being present between the motion sensor unit and the device.

In an embodiment, the motion sensor unit is mechanically connectable with the housing of the device. However, it represents a separate component that can be generally detached from the housing of the device. In this or another embodiment, the motion sensor unit is mechanically connected with the device during tracking the movement of the device. If the motion sensor unit is mechanically connected with the housing of the device, it forms part of the device for measuring the flow of gas. To give an example, a smart phone is an appropriate motion sensor unit that can be used to equip a device for measuring the flow of gas with a motion sensor. To avoid any inaccuracies that could result from a relative movement between the motion sensor unit and the housing (as well as other parts) of the device, it is important that the motion sensor unit is tightly connected with the housing of the device during calibration verification. Other external motion sensor units that can be generally used in connection with other devices are likewise suited to equip the assembly or the device with the motion sensor in order to enable the possibility of performing a calibration verification as explained above.

In an embodiment, the gas inlet and/or the gas outlet have a shape that serves for increasing the gas flow through the device and/or that reduces a dependency of the gas flow through the device on an orientation of the movement of the device through space in relation to an axis of a flow path through the device. A funnel-shaped inlet and/or a funnel-shaped outlet are examples of such shape serving at least for increasing gas flow through the device. By increasing the gas flow through the device and/or by reducing the dependency of the gas flow through the device on an orientation of the movement of the device through space in relation to an axis of a flow path through the device, the overall accuracy of measuring a gas flow and calculating the volume flowing through the device in a defined period of time can be generally increased.

In an embodiment, the shape of the gas inlet and/or the gas outlet is realized by placing a flow influencing device on the gas inlet and/or the gas outlet. The flow influencing device serves for increasing the gas flow through the device. In an embodiment, the flow influencing device has a funnel-like shape (i.e., a funnel-shaped appearance). The flow influencing device is connected to the gas inlet or the gas outlet during the calibration verification and is removed prior to performing gas flow measurements on a patient. The accuracy of the calibration verification is increased by using such a flow influencing device, however, it makes the calibration verification slightly less comfortable since it needs to be removed prior to performing gas flow measurements on a patient.

In an embodiment, the gas inlet and/or the gas outlet has (either due to its original shape or due to a connected flow influencing device) a shape having an opening angle (cone angle) lying in a range of from 5° to 120 °, in particular from 10° to 110°, in particular from 15° to 100°, in particular from 20° to 95°, in particular from 25° to 90°, in particular from 30° to 85°, in particular from 35° to 80°, in particular from 40° to 75°, in particular from 45° to 70°, in particular from 50° to 65°, in particular from 55° to 60°.

In an embodiment, the motion sensor is an accelerometer, an inertial measurement unit (IMU), a magnetometer, a light-based motion detector such as a laser-based motion detector, an acoustic motion sensor, and a radar-based motion sensor. Each of these examples of motion sensors is likewise appropriate to be used to equip the assembly, in particular the motion sensor unit or directly the device, with a motion sensor. A lidar-based motion detector (i.e., a specific light-based motion detector) is a particular appropriate example of the motion sensor if the motion sensor is arranged in the motion sensor unit. Such motion sensors are commercially available.

In an embodiment, a repetition of the calibration verification after the signal is generated due to fulfilling the second condition is performed only for a predeterminable limited number of times (such as 1 to 10 times, in particular 2 to 8 times, in particular 3 to 7 times, in particular 4 to 6 times, in particular 5 to 6 times) to avoid an endless loop of repeating calibration verifications in case that the device is indeed out of calibration.

In an embodiment, the first characteristic value is the detected gas flow, and the second characteristic value is the expected gas flow.

In an embodiment, the first characteristic value is the gas volume that has passed through the device during at least a part of the movement, in particular during the whole movement. This gas volume is calculated from the detected gas flow. In this embodiment, the second characteristic value is the gas volume that is expected to pass through the device during at least a part of the movement, in particular during the whole movement. This gas volume is calculated from the expected gas flow.

In an embodiment, the first characteristic value and the second characteristic value are curves representing the expected or detected gas flow over time during the movement of the device, respectively. In an embodiment, the first characteristic value and the second characteristic value are curves calculated from the expected or detected gas flow over time during the movement of the device, respectively. In such cases, the difference can be denoted as gain factor or scaling factor that is required to fit both curves onto each other. In an embodiment, the first characteristic value and the second characteristic value are a set of parameters of such curves.

If the assembly, in particular the device, comprises a processor and a memory unit, all required operations can be directly performed by the assembly or the device. This facilitates handling of the assembly or the device since no additional computing device is required. However, this embodiment requires a somewhat more complex construction of the assembly or the device. If, on the other hand, the assembly or the device is operatively connected with the processor and the memory unit, it only needs to be equipped with a data transmission unit that serves for transmitting the data obtained by the motion sensor to the processor for further evaluation. The evaluation itself is then done by a separate device that can be positioned remote from the device for measuring the flow of gas.

In an embodiment, the generated signal is presented to a user of the assembly on a surface of the assembly or the device, even in case that the signal is not generated by the assembly or the device itself, but rather by a remote processing device. In an embodiment, the generated signal is presented to the user on a display apparatus connected to the device and optionally also operatively connected to an evaluation unit such as a remote computing device. This display apparatus forms part of the assembly. In an embodiment, the display apparatus and/or a combination of the display apparatus and the evaluation unit is a smartphone.

In an embodiment, the computer-readable program causes the processor to consider a relative movement between gas (such as ambient air surrounding the device) and the device that is independent on any movement of the device in the space upon determining the second characteristic value. By such consideration, gas movements, e.g., due to wind or convection, can be appropriately compensated. Thus, any airflow not originating from a movement of the device, but being rather due to an inherent gas flow of the gas through which the device is moved upon the calibration verification procedure is then compensated for. This increases the accuracy of a calibration verification performed with the help of the motion sensor of the assembly.

In an embodiment, the computer-readable program causes the processor to store a progression of the absolute value of the difference over time in the memory unit. In an embodiment, the progression is presented to the user on the display apparatus. Then, it is possible for the user to evaluate the progression or course of the absolute value of the difference over time and to detect early signs for a (beginning) de-calibration. In an embodiment, the computer-readable program causes the processor to automatically evaluate the stored progression of the absolute value of the difference over time and optionally to automatically detect early signs for a (beginning) de-calibration. In an embodiment, a further signal is generated if a (beginning) de-calibration is detected to draw the user's attention to this fact of a (beginning) de-calibration of the device.

In an embodiment, the time during which the progression of the absolute value of the difference is stored is a time window covering 1 day to 1 year, in particular 2 days to 11 months, in particular 3 days to 10 months, in particular 4 days to 9 months, in particular 5 days to 8 months, in particular 6 days to 7 months, in particular 1 week to 6 months, in particular 2 weeks to 5 months, in particular 3 weeks to 4 months, in particular 4 weeks to 3 months, in particular 1 month to 2 months.

In an embodiment, the device is a spirometer, i.e., a device for assessing lung function in patients. Spirometers typically require a particularly accurate calibration verification to provide accurate measuring results for accurately determining the patient's lung function. Therefore, the provision of a motion sensor is particularly helpful in case of the device being a spirometer.

In an embodiment, the device is a spirometer and additionally implements the features described in EP 3 566 647 A1, i.e., the computer-readable program causes the processor to additionally perform the following steps: a) determining an actual value of a distance between a first ultrasonic transducer and a second ultrasonic transducer of the spirometer; b) determining a difference between the actual value of the distance and a nominal value of the distance that is assigned to the spirometer; and c1) accepting an actual calibration of the spirometer if an absolute value of the difference is smaller than or equal to a first threshold value, or c2) refusing an actual calibration of the spirometer if an absolute value of the difference is bigger than the first threshold value, wherein the first threshold value lies in a range from 0.5 % to 5 %, in particular from 1 % to 4 %, in particular from 2 % to 3 %, in particular from 1 % to 2 %, of the nominal value of the distance. Regarding all possible embodiments of such a spirometer, reference is made to EP 3 566 647 A1 which is incorporated herein by reference. All embodiments described in EP 3 566 647 A1 can also be applied to the presently claimed and described assembly and method. A combination of the present disclosure with the subject matter of EP 3 566 647 A1 provides the spirometer of EP 3 566 647 A1 with the possibility of measuring the flow of gas for performing a calibration verification. Thus, such a combination even enhances the accuracy of calibration verification since it relies on two different physical principles, namely, a distance measurement on the one hand and a measurement of actual flow through the device on the other hand.

In an aspect, the present invention relates to a method for verifying a calibration of a device for measuring the flow of gas, in particular of a device of an assembly according to the preceding explanations. The method comprises the steps explained in the following.

First, the device is moved through a space surrounding the device. Any movement by which the device is moved through the space such that gas is passed from a gas inlet of the device and/or a gas outlet of the device through the device is appropriate.

Upon moving the device, the resulting movement of the device through the space is detected with a motion sensor.

Furthermore, a gas flow through the device during the movement of the device is detected with a flow sensor of the device. Additionally, a first characteristic value that represents or is based on the detected gas flow is determined with a processor (either of the device or operatively coupled with the device).

In addition, the processor determines a second characteristic value that represents or is based on an expected gas flow corresponding to the detected movement of the device. This determination is done on the basis of the detected motion sensor data.

Furthermore, the processor is used to compare the second characteristic value with the first characteristic value. It is further used for determining an absolute value of a difference between the second characteristic value and the first characteristic value.

Subsequently, a first signal is generated if a first condition is met, and a second signal is generated if a second condition is met. According to the first condition, the absolute value of the difference between the second characteristic value and the first characteristic value is smaller than a predeterminable threshold. The first signal indicates that the calibration of the device still lies within a predeterminable range and is considered to be in order. According to the second condition, the absolute value of the difference between the second characteristic value and the first characteristic value is equal to or bigger than a predeterminable threshold. The second signal indicates that the calibration of the device is no longer in order. Thus, the generated signal indicates a status of the calibration verification and thus of the accuracy of the device and the measurements performed by the device.

In an embodiment, an amount of at least 50 %, in particular 50 % to 100 %, in particular 60 % to 95 %, in particular 70 % to 90 %, in particular 75 % to 80 %, of the movement of the device follows an axis of a flow path through the device (the flow path defining a connection between the gas inlet and the gas outlet of the device). In such a case it will be guaranteed in a particularly simple manner that the movement will produce a sufficient gas flow across the flow sensor.

In an embodiment, the device is moved with a speed lying in a range of from 0.1 m/s to 10 m/s, in particular from 0.2 m/s to 9 m/s, in particular from 0.3 m/s to 8 m/s, in particular from 0.4 m/s to 7 m/s, in particular from 0.5 m/s to 6 m/s, in particular from 0.6 m/s to 5 m/s, in particular from 0.7 m/s to 4 m/s, in particular from 0.8 m/s to 3 m/s, in particular from 0.9 m/s to 2 m/s, in particular from 1 m/s to 1.5 m/s. Such a speed is very appropriate to achieve a proper verification of calibration.

In an embodiment, the device is moved during a time period lying in a range from 1 s to 1 min, in particular from 2 s to 55 s, in particular from 3 s to 50 s, in particular from 4 s to 45 s, in particular from 5 s to 40 s, in particular from 6 s to 35 s, in particular from 7 s to 30 s, in particular from 8 s to 25 s, in particular from 9 s to 20 s, in particular from 10 s to 15 s. Such a time is fully sufficient and very appropriate to achieve a proper verification of calibration, in particular in combination with any of the before-mentioned movement speeds.

In an embodiment, moving the device is carried out by a waving or sinusoidal motion. Such a motion is particularly appropriate to allow a sufficiently high flow of gas through the device so that verification of calibration can be effected in a particularly efficient manner.

In an embodiment, moving the device is performed by a user of the device, e.g., according to a proposed movement pattern. In an embodiment, moving the device is performed by mechanical movement arrangement that comprises a fixing mechanism by which the device is fixed to the mechanical movement arrangement. Then, the movement can be performed according to a predefined movement pattern that is stored in a memory unit, such as a memory unit of the mechanical movement arrangement. Moving the device with a mechanical movement arrangement even increases the accuracy of the calibration verification since it can be guaranteed that the device is moved according to a predefined movement pattern that can include a specific amount of movements in different spatial directions, wherein any movement by which the device is moved through the space such that gas is passed from a gas inlet of the device and/or a gas outlet of the device through the device is appropriate.

In an embodiment, the method substitutes a calibration verification performed with a calibration syringe. Thus, the method is, in this embodiment, not performed in addition to the classic calibration verification done with the help of a calibration syringe, but rather as a stand-alone calibration verification method. This particularly simplifies the calibration verification of the device equipped with the motion sensor of forming part of the assembly comprising the motion sensor unit with the motion sensor.

Herewith disclosed is the use of a motion sensor for tracking movements of a device for measuring the flow of gas, in particular of a device of an assembly according to the above explanations, and for providing motion data to be used for verifying a calibration of the device for measuring the flow of gas. According to the inventors' knowledge, no such novel use of a motion sensor nor of the data provided by the motion sensor has been described in prior art. Thus, this use presents a novel application of motion sensors and the data provided by motion sensors. This novel use particularly facilitates the calibration verification of devices for measuring the flow of gas.

In an embodiment, the device the movements of which are tracked by the motion sensor is a spirometer. As explained above, spirometers require particularly accurate calibration verification so that the novel use of a motion sensor is particularly appropriate for spirometers.

All embodiments of the assembly can be combined in any desired way and can be transferred either individually or in any arbitrary combination to the described method and to the described use. Likewise, all embodiments of the described method can be combined in any desired way and can be transferred either individually or in any arbitrary combination to the described assembly and to the described use. Finally, all embodiments of the described use can be combined in any desired way and can be transferred either individually or in any arbitrary combination to the described assembly and to the described method.

Further details of aspects of the present invention will be explained in the following making reference to an exemplary embodiment and accompanying Figures. In the Figures:

Figure 1     schematically shows in a partially transparent view an embodiment of a device for measuring the flow of gas; and

Figure 2 schematically shows in a partially transparent view an embodiment of an assembly comprising a device for measuring the flow of gas.

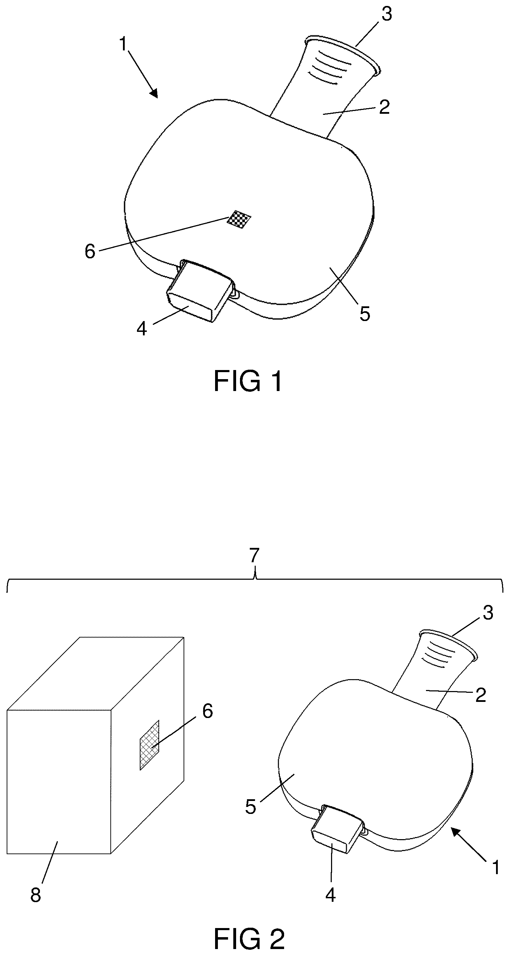

Figure 1 illustrates an embodiment of a spirometer 1 that serves as device for measuring the flow of gas. The spirometer 1 comprises a flow tube 2 having a gas inlet 3 and a gas outlet 4 opposite the gas inlet 3. During operation of the spirometer 1, a patient exhales breathing gas into the gas inlet 3. The exhaled breathing gas then flows through the flow tube 2 and exits the flow tube 2 at the gas outlet 4.

The spirometer 1 further comprises a housing 5 that comprises a flow sensor (not visible in Figure 1) that is arranged between the gas inlet 3 and the gas outlet 4. This flow sensor serves for measuring the flow of the breathing gas flowing through the flow tube 2.

A motion sensor 6 is arranged within the housing 5. For illustration purposes only, the housing 5 is illustrated transparent in the region of the motion sensor to allow a better view onto the motion sensor 6. With this motion sensor 6, all motions of the spirometer 1 can be accurately tracked.

To achieve a calibration verification of the spirometer 1, a user moves the spirometer 1 through the air such that air passes through the flow tube 2. In this context, it is of no importance if the air enters the flow tube 2 through the gas inlet 3 and exits the flow tube 2 through the gas outlet 4 or if it flows in opposite direction. A waving motion is a particularly appropriate motion of the spirometer 1. The motion should be repeated several times to obtain particularly reliable measurements. The motion sensor 6 transmits all available motion information to a processor located inside the housing 5. The processor can then derive the three-dimensional motion over time of the spirometer 1 in space.

The motion data is used for calculating a theoretical flow of air through the flow tube 2. This theoretical flow over time information (an example of the second characteristic value) can then be compared with the measured gas flow through the flow tube 2 (an example of the first characteristic value). If the absolute value of the difference between the theoretical flow and the measured flow is greater than a predeterminable threshold, the spirometer 1 is classified as "out of calibration". To provide a user of the spirometer 1 with the according information, a signal is generated indicating the "out of calibration" result of the comparison of the theoretical flow and measured flow. This signal can be presented to the user on a display provided on the housing 5.

As can be seen from Figure 1, the gas inlet 3 has a funnel-shaped appearance to improve the ratio between movement of the spirometer 1 and generated flow within the flow tube 2. This increases the accuracy of the calibration verification done with motion data provided by the motion sensor 6.

Figure 2 illustrates an embodiment of a spirometer assembly 7 comprising a spirometer 1 and a cradle 8. In this Figure, similar elements will be denoted with the same numeral references as in Figure 1.

The cradle 8 serves as motion sensor unit and comprises a motion sensor 6. With this motion sensor 6, it is possible to track movements of the spirometer 1. Thus, it is not necessary that the spirometer 1 itself comprises a motion sensor. Apart from this difference, the spirometer 1 is identical to the spirometer 1 shown in Figure 1. Therefore, reference is made to the explanations given above with respect to Figure 1 for further details of the spirometer 1 of the embodiment shown in Figure 2.

The data gathered by the motion sensor 6 of the cradle 8 is processed in the same way as explained above with respect to the embodiment shown in Figure 1. Thus, apart from the difference that the motion sensor 6 is not realized in the spirometer 1 but rather in the cradle 8 of the spirometer assembly 7, there is no relevant other difference between the embodiment shown in Figure 2 and the embodiment shown in Figure 1. In particular, the functionality of the spirometer assembly 7 is identical to the functionality of the spirometer shown in Figure 1.

Due to the presence of the cradle 8, it is possible to include a processor within the cradle 8 instead of within the spirometer 1. In any case, it is necessary to establish a data communication between the cradle 8 and the spirometer 1 to transfer flow data gathered by the spirometer 1 to the cradle 8 and/or motion data gathered by the motion sensor 6 of the cradle 8 to the spirometer 1. The flow data and motion data need to be combined in order to calculate an expected flow and to compare it with a measured flow to achieve a flow sensor calibration verification, as explained above with respect to the embodiment shown in Figure 1.

**Claims**

1. Assembly comprising a device for measuring a flow of gas, the device (1) comprising a gas inlet (3), a gas outlet (4), a flow sensor arranged between the gas inlet (3) and the gas outlet (4), and a motion sensor (6) for tracking a movement of the device (1), **characterized in that** the assembly (7) further comprises or is operatively connected with a processor and a memory unit, wherein the memory unit comprises computer-readable code that causes the processor to perform the following steps when being executed by the processor:

   a) detecting, with the motion sensor (6), a movement of the device (1) through a space surrounding the device (1);
   b) detecting, with the flow sensor, a gas flow through the device (1) during the movement of the device (1) and determining, with the processor, a first characteristic value that represents or is based on the detected gas flow;

c) determining, with the processor, a second characteristic value that represents or is based on an expected gas flow corresponding to the detected movement of the device (1);

d) comparing, with the processor, the second characteristic value with the first characteristic value and determining an absolute value of a difference between the second characteristic value and the first characteristic value;

e) i) generating a first signal if the absolute value of the difference between the second characteristic value and the first characteristic value is smaller than a predeterminable threshold, wherein the first signal indicates that a calibration of the device still lies within a predeterminable range and is considered to be in order, and/or ii) generating a second signal if the absolute value of the difference between the second characteristic value and the first characteristic value is equal to or larger than a predeterminable threshold, wherein the second signal indicates that the calibration of the device is no longer in order.

2. Assembly according to claim 1, **characterized in that** the motion sensor (6) is arranged on a housing (5) of the device (1) or within a housing (5) of the device (1).

3. Assembly according to claim 1, **characterized in that** the motion sensor (6) is arranged in a motion sensor unit (8) that is separate from the device (1).

4. Assembly according to claim 3, **characterized in that** that the motion sensor unit (8) is mechanically connectable with a housing (5) of the device (1) and is detachable from the housing (5) of the device (1).

5. Assembly according to any of the preceding claims, **characterized in that** at least one of the gas inlet (3) and the gas outlet (4) has a shape that serves for increasing a gas flow through the device (1) and/or that reduces a dependency of the gas flow through the device (1) on an orientation of the movement of the device (1) through space in relation to an axis of a flow path through the device (1).

6. Assembly according to any of the preceding claims, **characterized in that** the motion sensor (6) is chosen from the group consisting of an accelerometer, an inertial measurement unit, a magnetometer, a light-based motion detector, an acoustic motion sensor, and a radar-based motion sensor.

7. Assembly according to any of the preceding claims, **characterized in that** the computer-readable program causes the processor to consider a relative movement between gas and the device (1) that is independent on a movement of the device (1) in the space upon determining the second characteristic value.

8. Assembly according to any of the preceding claims, **characterized in that** the computer-readable program causes the processor to store a progression of the absolute value of the difference over time in the memory unit.

9. Assembly according to any of the preceding claims, **characterized in that** the device (1) is a spirometer.

10. Method for verifying a calibration of a device (1) for measuring a flow of gas, in particular of a device (1) of an assembly (7) according to any of the preceding claims, the method comprising the following steps:

a) moving the device (1) through a space surrounding the device (1);

b) detecting, with a motion sensor (6), a movement of the device (1) through the space;

c) detecting, with a flow sensor of the device (1), a gas flow through the device (1) during the movement of the device (1) and determining, with a processor, a first characteristic value that represents or is based on the detected gas flow;

d) determining, with the processor, a second characteristic value that represents or is based on an expected gas flow corresponding to the detected movement of the device (1);

e) comparing, with the processor, the second characteristic value with the first characteristic value and determining an absolute value of a difference between the second characteristic value and the first characteristic value;

f) i) generating a first signal if the absolute value of the difference between the second characteristic value and the first characteristic value is smaller than a predeterminable threshold, wherein the first signal indicates that a calibration of the device still lies within a predeterminable range and is considered to be in order, and/or ii) generating a second signal if the absolute value of the difference between the second characteristic value and the first characteristic value is equal to or larger than a predeterminable threshold, wherein the second signal indicates that the calibration of the device is no longer in order.

11. Method according to claim 10, **characterized in that** moving the device (1) is carried out by a waving or sinusoidal motion.

**Patentansprüche**

1. Anordnung mit einer Vorrichtung zur Messung eines Gasstroms, wobei die Vorrichtung (1) einen Gaseinlass (3), einen Gasauslass (4), einen zwischen dem Gaseinlass (3) und dem Gasauslass (4) angeordneten Strömungssensor und einen Bewegungssensor (6) zum Verfolgen einer Bewegung der Vorrichtung (1) umfasst, **dadurch gekennzeichnet, dass** die Anordnung (7) ferner einen Prozessor und eine Speichereinheit umfasst oder damit funktionsmäßig verbunden ist, wobei die Speichereinheit einen computerlesbaren Code umfasst, der bei Ausführung durch den Prozessor den Prozessor dazu veranlasst, die folgenden Schritte durchzuführen:

     a) Erfassen, mit dem Bewegungssensor (6), einer Bewegung der Vorrichtung (1) durch einen die Vorrichtung (1) umgebenden Raum;
     b) Erfassen, mit dem Strömungssensor, eines Gasstroms durch die Vorrichtung (1) während der Bewegung der Vorrichtung (1), und Ermitteln, mit dem Prozessor, eines ersten Kennwerts, der den erfassten Gasstrom repräsentiert oder darauf basiert;
     c) Bestimmen, mit dem Prozessor, eines zweiten Kennwerts, der einen erwarteten Gasstrom repräsentiert oder darauf basiert, der der erfassten Bewegung der Vorrichtung (1) entspricht;
     d) Vergleichen, mit dem Prozessor, des zweiten Kennwerts mit dem ersten Kennwert und Ermitteln eines Absolutwerts einer Differenz zwischen dem zweiten Kennwert und dem ersten Kennwert;
     e) i) Erzeugen eines ersten Signals, wenn der Absolutwert der Differenz zwischen dem zweiten Kennwert und dem ersten Kennwert kleiner ist als ein vorbestimmbarer Schwellenwert, wobei das erste Signal anzeigt, das eine Kalibrierung der Vorrichtung immer noch in einem vorbestimmbaren Bereich liegt und als in Ordnung angesehen wird, und/oder ii) Erzeugen eines zweiten Signals, wenn der Absolutwert der Differenz zwischen dem zweiten Kennwert und dem ersten Kennwert gleich oder größer ist als ein vorbestimmbarer Schwellenwert, wobei das zweite Signal anzeigt, dass die Kalibrierung der Vorrichtung nicht mehr in Ordnung ist.

2. Anordnung nach Anspruch 1, **dadurch gekennzeichnet, dass** der Bewegungssensor (6) an einem Gehäuse (5) der Vorrichtung (1) oder in einem Gehäuse (5) der Vorrichtung (1) angeordnet ist.

3. Anordnung nach Anspruch 1, **dadurch gekennzeichnet, dass** der Bewegungssensor (6) in einer Bewegungssensoreinheit (8) angeordnet ist, die von der Vorrichtung (1) getrennt ist.

4. Anordnung nach Anspruch 3, **dadurch gekennzeichnet, dass** die Bewegungssensoreinheit (8) mechanisch mit einem Gehäuse (5) der Vorrichtung (1) verbunden werden kann und von dem Gehäuse (5) der Vorrichtung (1) abgenommen werden kann.

5. Anordnung nach einem der vorhergehenden Ansprüche, **dadurch gekennzeichnet, dass** mindestens einer von dem Gaseinlass (3) und dem Gasauslass (4) eine Form hat, die dazu dient, einen Gasstrom durch die Vorrichtung (1) zu erhöhen, und/oder die eine Abhängigkeit des Gasstroms durch die Vorrichtung (1) von einer Ausrichtung der Bewegung der Vorrichtung (1) durch den Raum in Bezug auf eine Achse eines Strömungswegs durch die Vorrichtung (1) reduziert.

6. Anordnung nach einem der vorhergehenden Ansprüche, **dadurch gekennzeichnet, dass** der Bewegungssensor (6) aus der aus einem Beschleunigungsmesser, einer Trägheitsmesseinheit, einem Magnetometer, einem lichtbasierten Bewegungsmelder, einem akustischen Bewegungssensor und einem radarbasierten Bewegungssensor bestehenden Gruppe ausgewählt ist.

7. Anordnung nach einem der vorhergehenden Ansprüche, **dadurch gekennzeichnet, dass** das computerlesbare Programm den Prozessor dazu veranlasst, bei der Bestimmung des zweiten Kennwerts eine Relativbewegung zwischen dem Gas und der Vorrichtung (1) zu betrachten, die von einer Bewegung der Vorrichtung (1) im Raum unabhängig ist.

8. Anordnung nach einem der vorhergehenden Ansprüche, **dadurch gekennzeichnet, dass** das computerlesbare Programm den Prozessor dazu veranlasst, eine Progression des Absolutwerts der Differenz über die Zeit in der Speichereinheit zu speichern.

9. Anordnung nach einem der vorhergehenden Ansprüche, **dadurch gekennzeichnet, dass** die Vorrichtung (1) ein Spirometer ist.

10. Verfahren zur Überprüfung einer Kalibrierung einer Vorrichtung (1) zur Messung eines Gasstroms, insbesondere einer Vorrichtung (1) einer Anordnung (7) nach einem der vorhergehenden Ansprüche, wobei das Verfahren die folgenden Schritte umfasst:

     a) Bewegen der Vorrichtung (1) durch einen die Vorrichtung (1) umgebenden Raum;
     b) Erfassen, mit einem Bewegungssensor (6), einer Bewegung der Vorrichtung (1) durch den Raum;
     c) Erfassen, mit einem Strömungssensor der Vorrichtung (1), eines Gasstroms durch die Vorrichtung (1) während der Bewegung der Vorrichtung (1), und Ermitteln, mit einem Prozessor, eines ersten Kennwerts, der den erfassten Gasstrom repräsentiert oder darauf basiert;

d) Bestimmen, mit dem Prozessor, eines zweiten Kennwerts, der einen erwarteten Gasstrom repräsentiert oder darauf basiert, der der erfassten Bewegung der Vorrichtung (1) entspricht;

e) Vergleichen, mit dem Prozessor, des zweiten Kennwerts mit dem ersten Kennwert und Ermitteln eines Absolutwerts einer Differenz zwischen dem zweiten Kennwert und dem ersten Kennwert;

f) i) Erzeugen eines ersten Signals, wenn der Absolutwert der Differenz zwischen dem zweiten Kennwert und dem ersten Kennwert kleiner ist als ein vorbestimmbarer Schwellenwert, wobei das erste Signal anzeigt, das eine Kalibrierung der Vorrichtung immer noch in einem vorbestimmbaren Bereich liegt und als in Ordnung angesehen wird, und/oder ii) Erzeugen eines zweiten Signals, wenn der Absolutwert der Differenz zwischen dem zweiten Kennwert und dem ersten Kennwert gleich oder größer ist als ein vorbestimmbarer Schwellenwert, wobei das zweite Signal anzeigt, dass die Kalibrierung der Vorrichtung nicht mehr in Ordnung ist.

**11.** Verfahren nach Anspruch 10, **dadurch gekennzeichnet, dass** das Bewegen der Vorrichtung (1) durch eine wellenförmige oder sinusförmige Bewegung erfolgt.

## Revendications

**1.** Ensemble comprenant un dispositif de mesure d'un flux de gaz, le dispositif (1) comprenant une entrée de gaz (3), une sortie de gaz (4), un capteur de flux agencé entre l'entrée de gaz (3) et la sortie de gaz (4), et un capteur de mouvement (6) pour suivre un mouvement du dispositif (1),
**caractérisé**
**en ce que** l'ensemble (7) comprend en outre ou est relié de manière opérationnelle à un processeur et à une unité de mémoire, l'unité de mémoire comprenant un code lisible par ordinateur qui amène le processeur à effectuer les étapes suivantes lorsque le code est exécuté par le processeur :

a) détecter, à l'aide du capteur de mouvement (6), un mouvement du dispositif (1) dans un espace entourant le dispositif (1) ;
b) détecter, à l'aide du capteur de flux, un flux de gaz à travers le dispositif (1) pendant le mouvement du dispositif (1) et déterminer, à l'aide du processeur, une première valeur caractéristique qui représente ou est basée sur le flux de gaz détecté ;
c) déterminer, à l'aide du processeur, une deuxième valeur caractéristique qui représente ou est basée sur un flux de gaz attendu correspondant au mouvement détecté du dispositif (1) ;
d) comparer, à l'aide du processeur, la deuxième valeur caractéristique à la première valeur caractéristique et déterminer une valeur absolue d'une différence entre la deuxième valeur caractéristique et la première valeur caractéristique ;
e) i) générer un premier signal si la valeur absolue de la différence entre la deuxième valeur caractéristique et la première valeur caractéristique est inférieure à un seuil prédéterminable, le premier signal indiquant qu'un étalonnage du dispositif se situe encore dans une plage prédéterminable et est considéré comme étant en ordre, et/ou ii) générer un deuxième signal si la valeur absolue de la différence entre la deuxième valeur caractéristique et la première valeur caractéristique est égale ou supérieure à un seuil prédéterminable, le deuxième signal indiquant que l'étalonnage du dispositif n'est plus en ordre.

**2.** Ensemble selon la revendication 1, **caractérisé en ce que** le capteur de mouvement (6) est agencé sur un boîtier (5) du dispositif (1) ou à l'intérieur d'un boîtier (5) du dispositif (1).

**3.** Ensemble selon la revendication 1, **caractérisé en ce que** le capteur de mouvement (6) est agencé dans une unité de capteur de mouvement (8) qui est séparée du dispositif (1).

**4.** Ensemble selon la revendication 3, **caractérisé en ce que** l'unité de capteur de mouvement (8) peut être reliée mécaniquement à un boîtier (5) du dispositif (1) et peut être détachée du boîtier (5) du dispositif (1).

**5.** Ensemble selon l'une quelconque des revendications précédentes, **caractérisé en ce qu'**au moins l'une de l'entrée de gaz (3) et de la sortie de gaz (4) a une forme qui sert à augmenter un flux de gaz à travers le dispositif (1) et/ou qui réduit une dépendance du flux de gaz à travers le dispositif (1) à une orientation du mouvement du dispositif (1) à travers l'espace par rapport à un axe d'une voie d'écoulement à travers le dispositif (1).

**6.** Ensemble selon l'une quelconque des revendications précédentes, **caractérisé en ce que** le capteur de mouvement (6) est choisi dans le groupe constitué d'un accéléromètre, d'une unité de mesure inertielle, d'un magnétomètre, d'un détecteur de mouvement à base de lumière, d'un capteur de mouvement acoustique et d'un capteur de mouvement à base de radar.

7. Ensemble selon l'une quelconque des revendications précédentes, **caractérisé en ce que** le programme lisible par ordinateur amène le processeur à considérer un mouvement relatif entre le gaz et le dispositif (1) qui est indépendant d'un mouvement du dispositif (1) dans l'espace lors de la détermination de la deuxième valeur caractéristique.

8. Ensemble selon l'une quelconque des revendications précédentes, **caractérisé en ce que** le programme lisible par ordinateur amène le processeur à stocker une progression de la valeur absolue de la différence au fil du temps dans l'unité de mémoire.

9. Ensemble selon l'une quelconque des revendications précédentes, **caractérisé en ce que** le dispositif (1) est un spiromètre.

10. Procédé de vérification d'un étalonnage d'un dispositif (1) de mesure d'un flux de gaz, en particulier d'un dispositif (1) d'un ensemble (7) selon l'une quelconque des revendications précédentes, le procédé comprenant les étapes suivantes :

    a) déplacer le dispositif (1) dans un espace entourant le dispositif (1) ;
    a) détecter, à l'aide d'un capteur de mouvement (6), un mouvement du dispositif (1) à travers l'espace ;
    b) détecter, à l'aide d'un capteur de flux du dispositif (1), un flux de gaz à travers le dispositif (1) pendant le mouvement du dispositif (1) et déterminer, à l'aide d'un processeur, une première valeur caractéristique qui représente ou est basée sur le flux de gaz détecté ;
    c) déterminer, à l'aide du processeur, une deuxième valeur caractéristique qui représente ou est basée sur un flux de gaz attendu correspondant au mouvement détecté du dispositif (1) ;
    d) comparer, à l'aide du processeur, la deuxième valeur caractéristique à la première valeur caractéristique et déterminer une valeur absolue d'une différence entre la deuxième valeur caractéristique et la première valeur caractéristique ;
    e) i) générer un premier signal si la valeur absolue de la différence entre la deuxième valeur caractéristique et la première valeur caractéristique est inférieure à un seuil prédéterminable, le premier signal indiquant qu'un étalonnage du dispositif se situe encore dans une plage prédéterminable et est considéré comme étant en ordre, et/ou ii) générer un deuxième signal si la valeur absolue de la différence entre la deuxième valeur caractéristique et la première valeur caractéristique est égale ou supérieure à un seuil prédéterminable, le deuxième signal indiquant que l'étalonnage du dispositif n'est plus en ordre.

11. Procédé selon la revendication 10, **caractérisé en ce que** le déplacement du dispositif (1) est effectué par un mouvement ondulatoire ou sinusoïdal.

FIG 1

FIG 2

**EP 4 591 788 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3566647 A1
- CN 109009130 A
- US 20190254534 A1
- US 2021338104 A1

**Non-patent literature cited in the description**

- **GRAHAM BL ; STEENBRUGGEN I ; MILLER MR et al.** Standardization of Spirometry 2019 Update. *Am J Respir Crit Care Med*, 2019, vol. 200 (8), e70-e88